Europäisches Patentamt

**European Patent Office**

**Office européen des brevets**

(19)

(11) Publication number: **0 124 874**
A2

(12) # EUROPEAN PATENT APPLICATION

(21) Application number: **84104967.9**

(22) Date of filing: **03.05.84**

(51) Int. Cl.³: **C 12 N 15/00**
C 12 P 21/02, C 12 N 1/18
//(C12N1/18, C12R1/865),
(C12P21/02, C12R1/865)

(30) Priority: **05.05.83 US 491672**

(43) Date of publication of application:
**14.11.84 Bulletin 84/46**

(84) Designated Contracting States:
**AT BE CH DE FR GB IT LI LU NL SE**

(71) Applicant: **F. HOFFMANN-LA ROCHE & CO.**
**Aktiengesellschaft**

**CH-4002 Basel(CH)**

(72) Inventor: **Kramer, Richard**
**9 Edgewood Avenue North**
**West Orange, N.J. 07052(US)**

(74) Representative: **Lederer, Franz, Dr. et al,**
**Patentanwälte Dr. Franz Lederer Dipl.-Ing. Reiner F.**
**Meyer-Roxlau Lucile-Grahn-Strasse 22**
**D-8000 München 80(DE)**

(54) **Novel vectors for interferon expression.**

(57) Methods for producing interferon with transformed yeast organisms wherein expression of the interferon genes is regulated via the concentration of inorganic phosphate in the growth medium or the temperature, the expression vectors and transformed host organisms useful therefore.

FIG. 5

EP 0 124 874 A2

F. Hoffmann-La Roche & Co. Aktiengesellschaft  **0124874**

Basel / Schweiz                                    3. Mai 1984

RAN 4100/35

## Novel vectors for interferon expression

Recent advances in molecular biology have made it possible to introduce DNA sequences coding for specific proteins from higher organisms into yeast cells. Recombinant DNA technology has, in fact, been used to express eucaryotic proteins, whose genes have been cloned in vectors, such as plasmids, in yeast cells, typically Saccharomyces cerevisiae which have been transformed with those vectors. See Hitzeman, R. A., et al., (1981) Nature $\underline{293}$, 717-722; Valenzuela, P., et al., (1982) Nature $\underline{298}$, 347-350; Tuite, M. F., et al., (1982) EMBO Journal $\underline{1}$, 603-608.

The expression of heterologous genes in S. cerevisiae achieved by Hitzeman et al. and Valenzuela et al. was constitutive (or continuous) because the genes were linked to the promoter region from the unregulated alcohol dehydrogenase (ADH) I gene. Tuite et al. and Hitzeman et al., (1983) Science $\underline{219}$, 620-625, used the promoter from the phosphoglycerate kinase gene for expression of human interferon genes.

The prior art already recognized that regulated synthesis of a foreign gene would be desirable because over-production of even normally non-toxic gene products may be harmful to host cells and lead to decreased stability of particular host-vector systems. A good expression control sequence, therefore, in addition to improving the efficiency of transcription and translation of cloned genes, should be controllable so as to modulate expression during host cell growth. Desired expression control sequences are those that may be switched off to enable the host cells to

Mez/11.4.84

propagate without build-up of the desired foreign gene products and then to be switched on to promote the expression of large amounts of the desired protein products. Tuite et al., using the PGK gene promoter, control the level of interferon expression by the carbon source. While the "induced" level of interferon expression was high, the "uninduced" level was only moderately high, resulting in an induction of less than 20-fold. Miyanohara, A. et al., (1983) Proc. Natl. Acad. Sci. USA $\underline{80}$, 1-5, have reported the regulated expression of heptatitis B virus surface antigen in yeast with the yeast acid phosphatase gene (PHO5) promoter.

The prior art, however, lacked a tightly regulated control element for the expression of interferon genes in yeast cells in which the "uninduced" level of foreign interferon protein expression was low and the "induced" level was high. It is, therefore, an object of this invention to provide a regulatory control element of this type which would allow for the growth of yeast cells to a high population density without concurrently risking a harmful buildup of the interferon protein until it is actually desired to have the yeast cell induce and express the interferon protein.

The present invention provides an improved expression vector capable of regulating the expression of heterologous genes in a transformed yeast strain. More specifically, this improved expression vector which incorporates the promoter/regulator DNA region from the yeast repressible acid phosphatase gene, regulates the expression of the gene for human leukocyte interferon.

This invention also provides for yeast cells transformed with the improved expression vector. These transformed yeast cells produce significant amounts of interferon only when grown in medium lacking inorganic phosphate ($P_i$).

This invention further provides for transformed yeast cells which are also mutants in two acid phosphatase regulatory genes (coding for a defective repressor and a temperature-sensitive positive regulator) which grow well at a high temperature (30-35°C) but produce foreign proteins, i.e. interferon, only at a low temperature (20-30°C), independent of phosphate concentration.

## Brief Description of the Drawings

Figure 1 is a map of the PHO5 promoter 5'-flanking region. The RNA 5'-end position and the translation initiation codon as well as relevant restriction sites for the PHO5 gene are shown. The Taq I site (at position -11 from the ATG) between the RNA 5'-end and the ATG was converted to an EcoRI site for the expression vector. The major RNA 5'-ends for PHO5 transcripts map at positions -41 and -35.

Figure 2A illustrates the pYE4 expression vector. The ClaI to EcoRI fragment with the PHO5 transcription start site was inserted into the yeast/E. coli shuttle vector YRp7 (11) that had the EcoRI site in parentheses deleted. In addition the 1.1 Kb ClaI fragment from the PHO5 promoter (fig. 1) and sequences from the yeast 2 micron plasmid were added. The gene to be expressed under PHO5 type regulation is inserted at the remaining EcoRI site. ( ⬜ = pBR322 DNA, ⬛ = yeast DNA, ▨ = 2 micron plasmid DNA).

Figure 2B illustrates the nucleotide sequences preceding the translation initiation codon ATG in the original PHO5 gene and in the PHO5/rIFN-αD fusion.

Figure 3 schematically illustrates the construction of pYE4.

Figure 4 schematically illustrates the construction of a plasmid used to construct pYE4.

Figure 5 illustrates the plasmid pYE4 with the relevant restriction sites indicated.

Figure 6 illustrates the plasmid pYE7 with the relevant restriction sites indicated.

Figure 7 graphically illustrates the regulated expression of rIFN-αD in yeast. Transformants carrying pYE4-D were grown in high-$P_i$ medium (solid symbols) or no-Pi medium (open symbols). The cell growth (●,○), APase (acid phosphatase) activity (■,□) and interferon activity in extracts (▲,△) were monitored. T = 0 is the time at which the starter culture was divided into high-$P_i$ or no-$P_i$ medium.

Figure 8 illustrates interferon RNA levels. RNA samples were prepared from the cells described in Figure 3 (+ = high-$P_i$, - = no-$P_i$) at the indicated times and analyzed by gel blot analysis (17).

Figure 9 graphically illustrates the temperature regulation of interferon synthesis. The pho80 pho4[ts] strain 29B5 transformed with pYE4-D was grown at 35°C to an $OD_{600nm}$ of 1, at which time (t = 0) half the culture was shifted to 23°C (open symbols) and the other half was kept at 35°C (solid symbols). Cell growth (●,○), A Phase activity (■,□) and interferon activity (▲,△) were monitored.

The vector system of the present invention, which tightly regulates expression of foreign genes in yeast, utilizes the 5'-flanking region from the yeast repressible acid phosphatase (APase) gene, otherwise known as the PHO5 gene. Transcription of the PHO5 gene in a yeast cell is repressed when inorganic phosphate ($P_i$) is present in the growth medium but is induced to a high level when $P_i$ is depleted. A unique restriction enzyme site was introduced downstream from the transcription start site but upstream from the ATG initiator codon on a DNA fragment carrying the putative promoter/regulatory DNA region from the PHO5 gene. A structural gene inserted into this site will have translation started at its own initiator codon, resulting in an authentic or mature rather than a fusion protein. The structural gene used with this invention is preferably a eucaryotic gene which encodes for a protein foreign to that of the host yeast cell. The particular eucaryotic genes used in this invention are those which encode for mature human leukocyte interferons, especially A and D species (IFN-αA and IFN-αD). The gene sequences which encode for these proteins and their corresponding amino acid sequences are known and have been described in various printed publications. See Pestka, "The Human Interferons – From Protein Purification and Sequence to Cloning and Expression in Bacteria: Before, Between and Beyond", Archives of Biochemistry and Biophysics, 221, No. 1, 1-37 (1983). Although human eucaryotic genes were used in this invention, it is contemplated that gene from any eucaryotic species (e.g. bovine, murine and porcine) could also be used with the PHO5 gene promoter/regulator system. However, as described in the examples and specification which follows the PHO5 gene promoter expression system of this invention was tested by inserting the cloned genes for human leukocyte interferon rIFN-αD and rIFN-αA into an expression vector.

Another efficient and preferred method for induction, especially for large-scale growth, utilizes a strain of Saccharomyces cerevisiae APase regulatory mutants (8) to develop a yeast strain that induces APase and, therefore, genes in the PHO5 promoter expression vector (which vector includes regulatory and structural genes) only in response to temperature. At the non-permissive temperature (about 30-35°C) the cells grow well but produce no APase, even in low-$P_i$ medium, while at the permissive temperature (about 20-30°C, preferably 23°C) APase is synthesized with or

without $P_i$ in the medium. This strain carrying the PHO5 promoter expression vector has also be used to demonstrate temperature regulated expression of foreign genes specifically the rIFN-αD gene in yeast. The production of leukocyte interferon is induced at the lower temperatures, preferably 23°C.

The materials utilized in the various embodiments of this invention are described below. The reference numbers in parentheses included throughout the specification correspond to the references cited in the attached bibliography.

Yeast Strains and Media:

W301-18A (αade2-1 leu2-3, 112 trp-1 can1-100 ura3-1 his3-11,15) was obtained from R. Rothstein. A138 (a PHO5-2 pho80-2) was obtained from P. Hansche (9) and R6-3A (αpho4$^{ts}$) was from A. Toh-e (Tohe et al., (1981) J. Bact. 145, 221-232). Strains from this work are P1-22 (a pho80 trp1 ade2 his3 leu2), 29B5 (a pho80 pho4$^{ts}$ trp1 ade2 his3 leu2) and 29A21 (α pho80 trp1 ade2 leu2). One skilled in the art whould be able, in the practice of this invention, to substitute equivalent yeast strains. In the case of W301-18A another yeast strain may be used which has the trp1 marker. Any pho4$^{ts}$ or pho80 mutant yeast strain may also be used in the practice of the invention.

High $P_i$ medium was YNB+CAA ("YNB" - yeast nitrogen base; "CAA" - casamino acids) (1) with adenine and uracil added when required. No-$P_i$ medium was UMD (6) plus uracil when required with no added inorganic phosphate. Both mediums lack tryptophan for selection of transformants.

DNA and Plasmmids:

The PHO5 gene was obtained from an 8 kilobase (kb) EcoRI restriction fragment shown to contain this gene (7,10). The yeast/E. coli shuttle vector, YRp12, was from R. Davis (Scherer and Davis [1979] Proc. Nat. Acad. Sci. 76, 4951-4955). The 0.56 kb EcoRI fragment carrying rIFN-αD was obtained from Genentech, Inc., on the plasmid pFRS36 (1). The yeast gene for glyceraldehyde 3-phosphate dehydrogenase was obtained by screening a yeast DNA library with the cloned chicken gene.

Construction of Recombinant Plasmids:

Restriction endonucleases were from New England Biolabs and Boehringer-Mannheim Biochemical, and $T_4$ DNA ligase was from New England Biolabs. The enzymes were used as recommended by the suppliers. Synthetic BamHI and EcoRI linkers were from Collaborative Research.

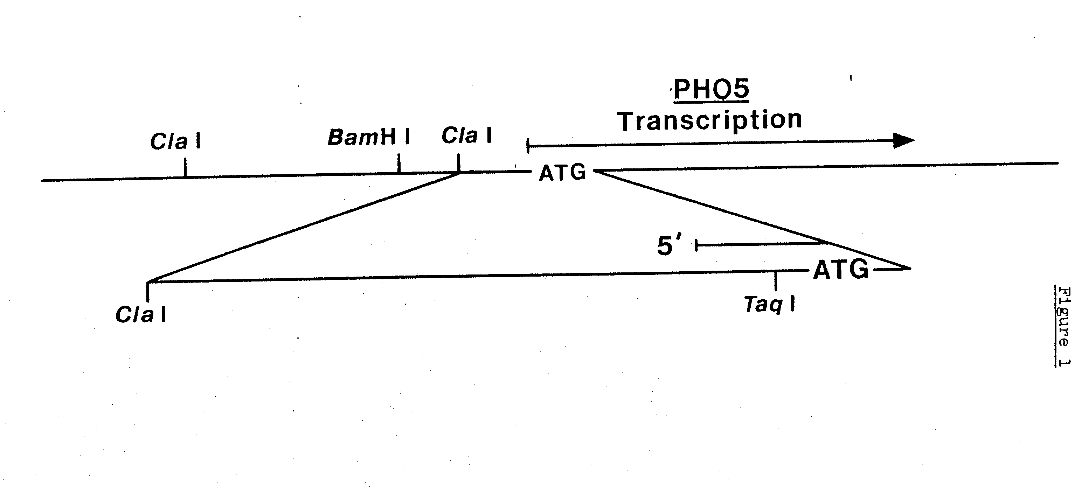

Generally stated, in order to construct a PHO5 promoter expression vector a unique restriction enzyme site was introduced into the DNA region between the position of the 5'-end of the PHO5 transcript (18) and the initiator ATG by converting the TaqI site, shown in Figure 1, to an EcoRI site with a synthetic oligonucleotide linker.

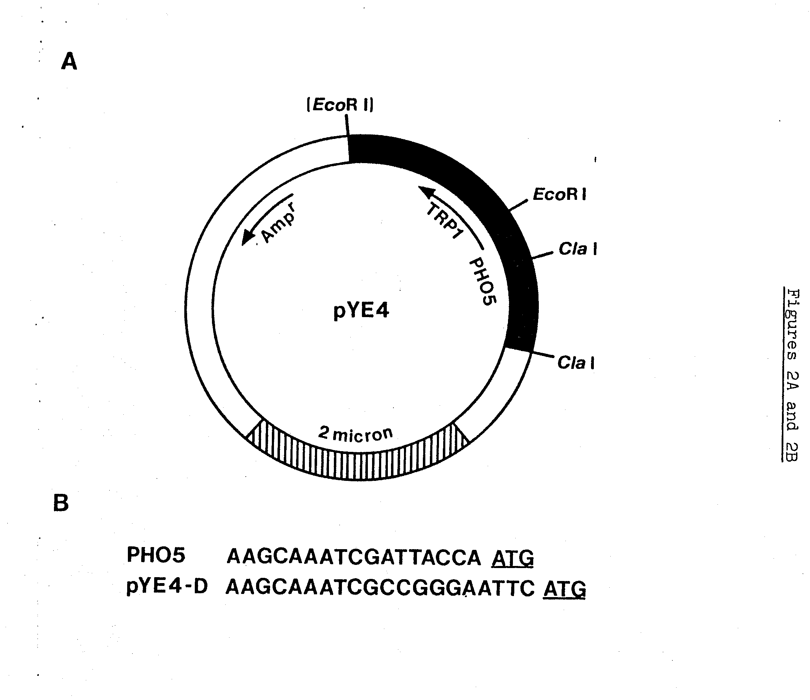

The resulting ClaI to EcoRI fragment with the transcription start site was inserted into the vector pBR322 (6) along with the yeast TRP1 gene on a 1.4 kb EcoRI restriction fragment resulting in a plasmid similar to YRp7 (11) with the PHO5 promoter. Because DNA sequences upstream from the ClaI site are required for full expression and regulation of the PHO5 gene, the additional ClaI fragment (Figure 1) was inserted, and a segment of DNA from the naturally occurring yeast 2 micron plasmid (20) was added to increase copy number and stability of the vector. Finally, one EcoRI site (indicated in Fig. 1) was eliminated. The resulting plasmid, pYE4, is diagrammed in Figure 2A. A cloned gene carrying an ATG initiation codon was inserted into the single EcoRI site in pYE4. Phosphate regulated transcription initiated in the PHO5 promoter, proceeded through the inserted gene and terminated either in the 3' untranslated region of the inserted DNA or at the termination site for the TRP1 gene.

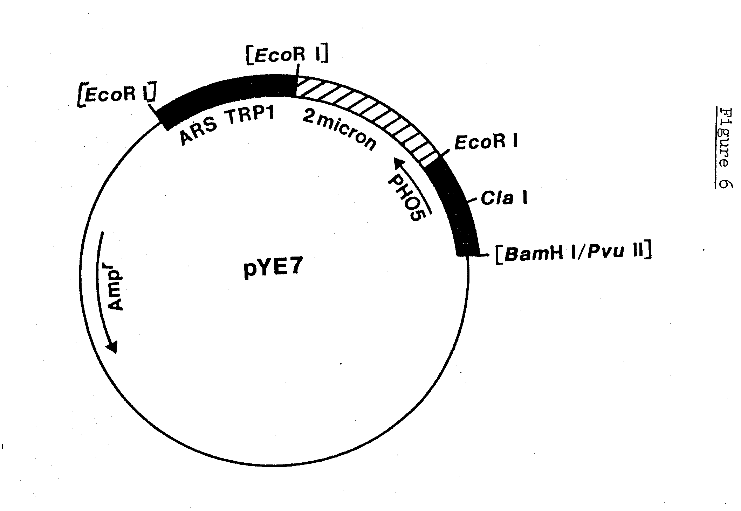

Another plasmid, pYE7, was constructed in a similar manner (Fig. 6). In this case, the 2 micron DNA was inserted between the PHO5 promoter and the TRP1 fragment, and the EcoRI site distal to the promoter was again deleted. In addition, the pBR322 sequences from the BamHI site to the PvuII site (1690 base-pairs) were eliminated.

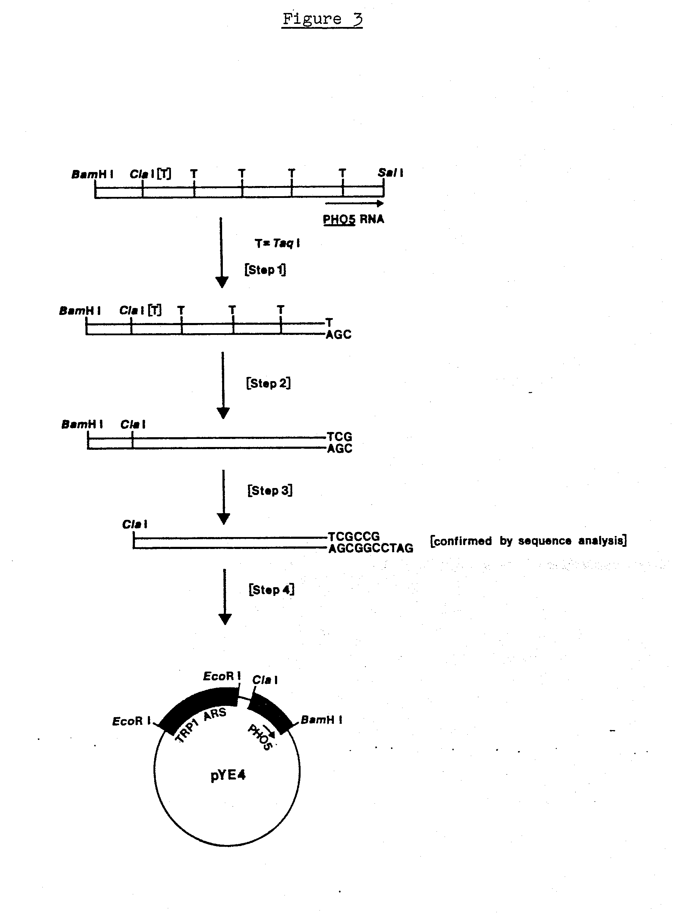

More specifically, the PHO5 promoter expression vectors were constructed as described below. The BamHI to SalI fragment with the PHO5 promoter (7,8) was prepared from an 8 kb EcoRI fragment isolated previously (7) (Figure 3).

The BamHI to SalI fragment was partially digested with TaqI and a 540 bp BamHI/TaqI fragment was isolated (step 1, Figure 3). The TaqI "sticky end" was filled in with E. coli DNA polymerase large fragment (Klenow fragment) and dCTP and dGTP (step 2, Figure 3). This fragment was then ligated with BamHI linker (CCGGAATTCCGG) and cut with BamHI and ClaI (step 3, Figure 3). The 270 bp ClaI/BamHI fragment was then ligated into a BamHI/ClaI cut plasmid YRp12 (Scherer and Davis [1979] Proc. Nat. Acad. Sci. 76, 4951-4955) (step 4, Figure 3).

Next, DNA from pYE1 was cut with BamHI, treated with S1 nuclease to remove the BamHI "sticky ends" and an EcoRI linker (GGAATTCC) was ligated on. The DNA was then cut with ClaI and EcoRI and the resulting 270 bp fragment with the PHO5 promoter was ligated into pBR322 (19) cut with ClaI and EcoRI.

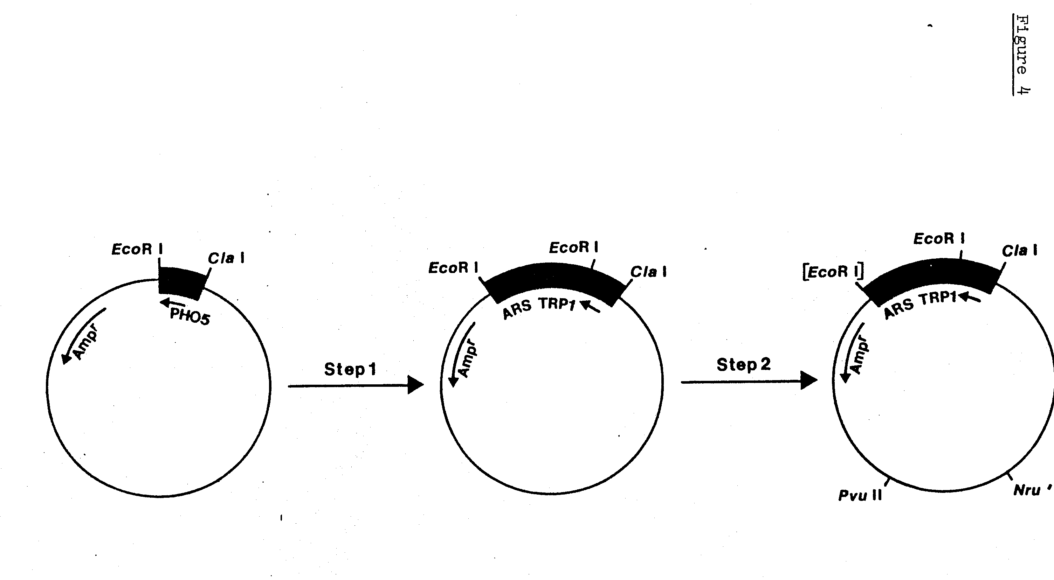

The resulting plasmid (Fig. 4) was then cut with EcoRI and the 1.4 kb EcoRI from YRp12 (with the yeast TRP1 gene and an ARS (replication origin) was ligated in (step 1, Figure 4).

The resultant plasmid was then partially digested with EcoRI and plasmids in which only one EcoRI site had been cut were isolated. The EcoRI sticky ends were filled in with DNA polymerase Klenow fragment and dATP and dTTP. After ligation of the DNA, plasmids missing the EcoRI site shown in () were selected (step 2, Figure 4).

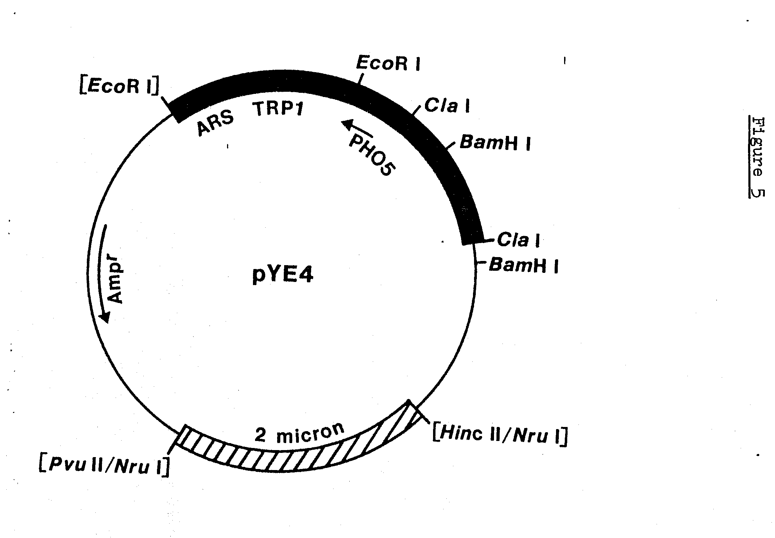

Next, the plasmid DNA was cut with PVUII and NruI and an 1875 bp NruI to HincII fragment of the yeast 2 micron plasmid (20) from YEp24 (Botstein et al. (1979) Gene 8, 17-24) was inserted. The resulting plasmid was cut with ClaI and the 1.1 kb ClaI fragment from the upstream regi n of the PHO5 promoter (13,18) was inserted to reconstruct the entire PHO5 promoter/regulatory region. The resulting plasmid was pYE4 (Figure 5).

The vector pYE7 was constructed in a manner similar to pYE4 except that the 2 micron DNA was a 2.2 kb EcoRI fragment inserted into the EcoRI site. The promoter distal EcoRI site was removed as for pYE4. The plasmid was then cut with BamHI and the sticky ends filled in with DNA polymerase Klenow fragment. After cutting with PvuII, the remaining plasmid was ligated to form pYE7, shown in Figure 6. Note that the ligation of a filled in BamHI site and a PvuII site recreated the BamHI recognition site.

To test these vectors, the 560 bp EcoRI fragment with rIFN-αD (1) was first inserted in the correct orientation at the unique EcoRI site of pYE4 and a plasmid carrying the rIFN-αD gene in the correct orientation for expression from the PHO5 promoter, designated pYE4-D, was obtained. Figure 2B compares the nucleotide sequences of the 5'-noncoding regions of the original PHO5 gene and of the PHO5/rIFN-αD fusion in pYE4-D. The extra CCGG sequence in pYE4-D between the former TaqI site (TCGA) and the EcoRI site (GAATTC) is from the synthetic linkers.

Yeast cells were then transformed with pYE4-D by methods known to those skilled in the art for transforming yeast cells with plasmids.

Generally stated, the preferred procedure for producing interferon, therefore, comprises:

a)  transforming a yeast organism with an expression vector containing the structural gene for a human interferon, preferably a leukocyte interferon, operably linked to a PHO5 promoter/regulator;

b)  cloning the transformed organism in a culture medium containing inorganic phosphate and essential nutrients for growth of the organism;

c)  decreasing the levels of inorganic phosphate present in the culture medium until said transformed organism is induced to produce interferon;

d)  lysing the transformed organism, and

e)  recovering interferon from the resultant lysate.

As will be further described in the examples, the yeast transformants were grown at 30°C in high-$P_i$ medium to a density of about $3 \times 10^6$ cells/ml ($OD_{600\ nm} = 0.5$) at which time half of the culture was removed and these cells were harvested by centrifugation, washed with sterile $H_2O$ and resuspended in no-$P_i$ medium. Growth was continued at about 30°C and samples were removed at approximately 3 hour intervals for measurement of cell density ($OD_{600\ nm}$) and APase activity (13) expressed as $A_{420\ nm}$ units from a 30 minute assay per 1 $OD_{600\ nm}$ unit of cells. Cell extracts for RNA (14) and interferon were also prepared.

An alternative preferred procedure for producing interferon comprises:

a)   transforming a temperature sensitive yeast organism with an expression vector containing the structural gene for a human interferon, preferably a leukocyte interferon, operably linked to a PHO5 promoter/regulator;

b)   cloning the transformed organism in a culture medium at a temperature of about 30-35°C;

c)   decreasing the temperature of the organism to about 20-30°C whereby interferon production is induced;

d)   lysing the transformed organism, and

e)   recovering interferon from the resultant lysate.

It is preferred that the temperature sensitive yeast organism is an acid phosphatase regulatory mutant strain of Saccharomyces cerevisiae.

As will be further described in the examples, the temperature shift transformants of this invention were grown in high-$P_i$ medium at about 35°C to an $OD_{600\ nm}$ of about 1. The culture was then divided in half. One aliquot was continued at 35°C while the other was grown at 23°C. Samples were removed at various times and prepared for analysis.

For each time point, approximately $10^8$ cells were harvested by centrifugation and resuspended in 1 ml of 7M guanidine-HCl, 1 mM dithiothreitol and 1 mM phenylmethylsulphonyl fluoride. An equal volume of acid washed glass beads was added, and the samples were vortexed for four 30 sec bursts. After 30 minutes on ice, cell debris and glass beads were removed by centrifugation, and the samples

- 12 -

were stored at -20°C. For interferon assay, the samples were either diluted 1:100 into 0.15 M NaCl, 20 mM $NaPO_4$, pH 7.9 (PBS), or dialyzed into PBS. Interferon activity was detected by the reduction of cytopathic effect assay (15). Activities are expressed in units per liter of culture of an $OD_{600\ nm}$ of 1.

Total RNA from each time point was subjected to electrophoresis in 1.4% agarose gels after denaturation with glyoxal (16) and transferred to nitrocellulose (17). Hybridizations were carried out as described in (13) with $^{32}$P-labeled probes.

## Example 1

Regulation of interferon synthesis by phosphate concentration.

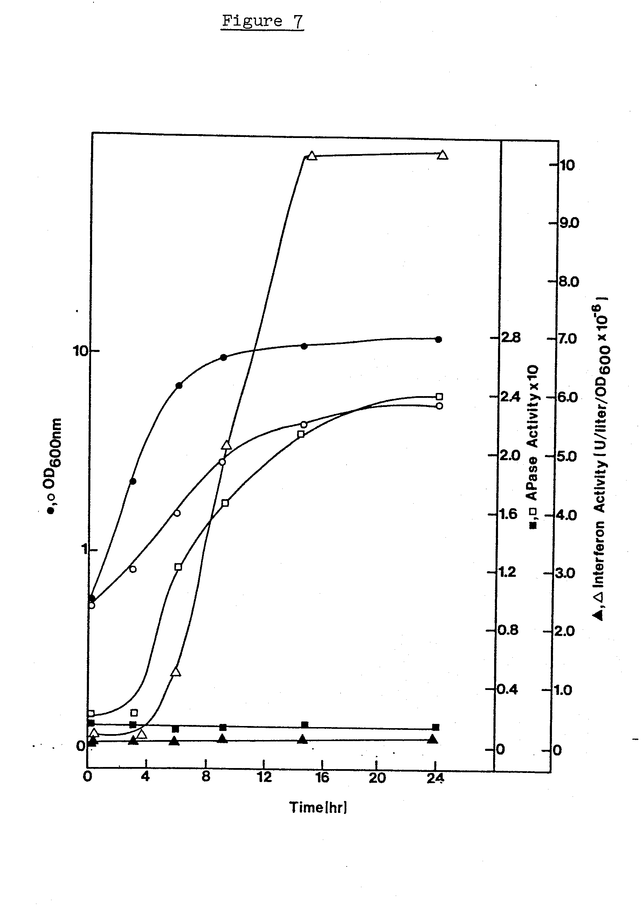

Trp[+] transformants of W301-18A carrying pYE4-D were grown in high-$P_i$ medium at 30°C to an $OD_{600 \ nm}$ of about 0.5 at which time half of the culture was transfered to no-$P_i$ medium. At approximately 3 hours intervals, aliquots of each culture were removed and assayed for APase activity and extracts were prepared for interferon assay. Figure 7 shows that the level of interferon activity began to increase rapidly between 3 and 6 hours after transfer to no-$P_i$ medium and peaked at around 9 hours. This induction corresponded to the APase induction observed for the culture and represented a 100- to 200-fold increase over the interferon activity detected in cells grown in high-$P_i$ medium.

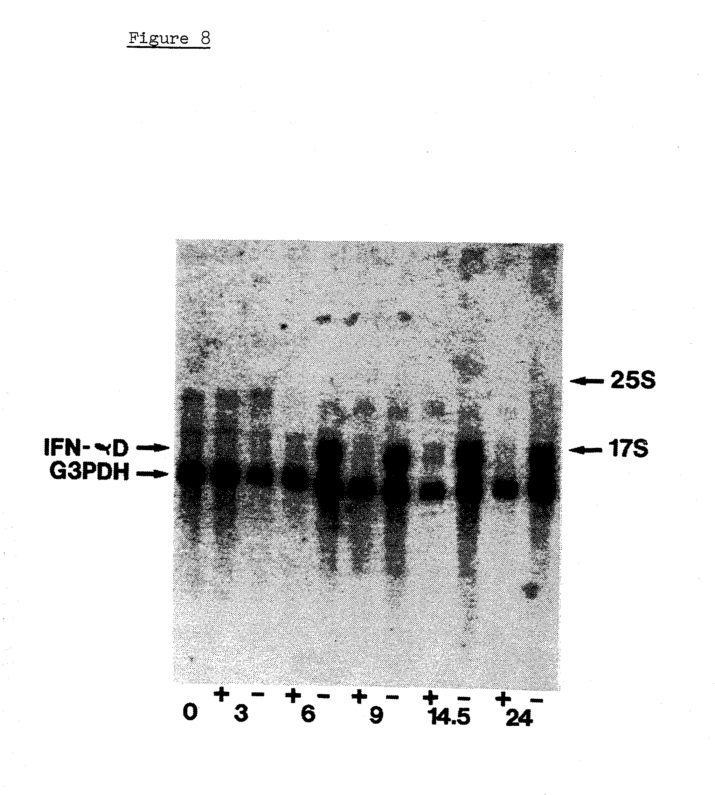

RNA samples were prepared from both cultures at the same times that the interferon extracts were made. Following denaturation with glyoxal (16), the RNA was fractionated on an agarose gel and then transfered to nitrocellulose (17). Figure 8 shows the results of hybridization of the RNA blot with labeled rIFN-αD probe. The appearance of rIFN-αD specific transcript corresponds to the appearance of interferon activity observed in the extracts. Thus, the regulation of interferon synthesis seen here is at the level of transcription as is the regulation of APase (6,7). In addition, the size of the rIFN-αD specific RNA is about 1.5 kb, the size expected for a transcript starting in the PHO5 promoter, going through the rIFN-αD gene and terminating at the end of the TRP1 gene.

Levels of up to 2 to 3 x 10[7] units of interferon activity per liter of culture at an $OD_{600 \ nm}$ of 1 were obtained.

## Example 2

Regulation of interferon synthesis by temperature.

To construct a yeast strain capable of temperature regulated APase induction, it was first desirable to eliminate the requirement for induction by low-$P_i$. Yeast mutants in the PHO80 (formerly known as PHOR) gene, a repressor of APase synthesis, produce APase constitutively (9,21). Therefore, a pho80 strain (A138) was crossed with the trp1 strain (W301-18A) used in Example 1. A pho80 trp1 strain (P1-22) was obtained and shown to produce APase in high-$P_i$ medium. P1-22 was then crossed with a strain with a temperature sensitive mutation in the PHO4 (formerly PHOD) gene (R6-3A). PHO4 codes for a positive regulator of APase synthesis (21) and, therefore, pho4 strains are unable to produce APase even in low-$P_i$. A temperature sensitive pho4 strain can be induced for APase only at the permissive temperature.

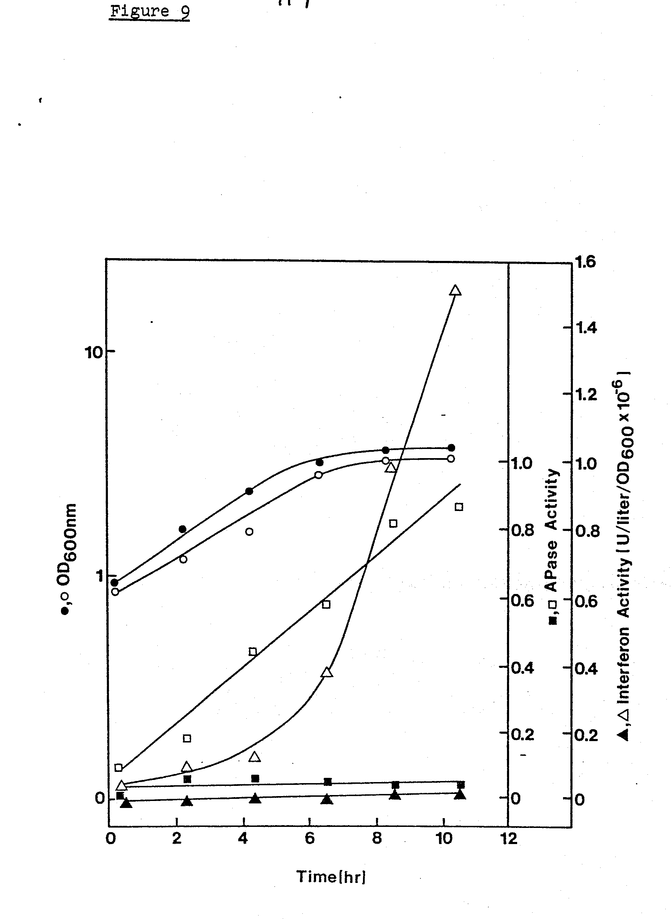

A pho4$^{ts}$ pho80 trp1 host (29B5) was transformed with pYE4-D, and transformants were tested for temperature regulated interferon synthesis. Cells were grown at 35°C to an $OD_{600\ nm}$ of about 1, and the culture was split into two portions. One continued to grow at 35°C while the other was shifted to the permissive temperature (23°C). At approximately 2 hour intervals, aliquots were removed and prepared for interferon assay. Figure 9 shows that interferon activity was at least 50 times higher in the cells grown at 23°C.

Significant interferon activity is observed only at the permissive temperature and the induced level reaches a maximum about 2 times higher than the final point in Figure 5, i.e., about $3 \times 10^6$ units per liter per $OD_{600\ nm}$.

## Example 3

<u>Synthesis of interferon in yeast with pYE7.</u>

The 560 bp EcoRI fragment with the rIFN-αD gene was inserted into the EcoRI site of pYE7 in the correct orientation to form pYE7-D. Alternatively, the rIFN-αA gene (Goeddel et al., (1980) Nature <u>287</u>, 411-415) on an 865 bp EcoRI/PstI fragment was inserted into the EcoRI site of pYE7 after converting the PstI site to an EcoRI site with a synthetic oligonucleotide linker. The resulting plasmid with the rIFN-αA gene in the correct orientation relative to the promoter was designated pYE7-A.

The plasmids pYE7-A and pYE7-D were transformed into yeast strain W301-18A, and $Trp^+$ transformants of each were induced as in Example 1. Samples were assayed for IFN activity at about 8 hours after transfer to no-$P_i$ medium. Levels of 5 to 7.5 x $10^7$ units of activity per liter per $OD_{600\ nm}$ units were obtained for pYE7-D and 1.0 to 1.5 x $10^8$ units/liter/$OD_{600\ nm}$ for pYE7-A. The somewhat higher level of rIFN-αA is probably due to the slightly higher specific activity of rIFN-αA in the assay system.

# BIBLIOGRAPHY

1. Hitzeman, R. A., Hagie, F. E., Levine, H. L., Goeddel, D. V., Ammerer, G., and Hall, B. D. (1981) Nature 293, 717-722.

2. Valenzuela, P., Medina, A., Rutter, W.J., Ammerer, G. and Hall, B. D. (1982) Nature 298, 347-350.

3. Tuite, M. F., Dobson, M. J., Roberts, N. A., King, R. M., Burke, D. C., Kingsman, S. M. and Kingsman, A. J. (1982) EMBO Journal 1, 603-608.

4. Schurr, A. and Yagil, E. (1971) J. Gen. Microbiol. 65, 291-303.

5. Toh-e, A., Kakimoto, S. and Oshima, Y. (1975) Molec. Gen. Genet. 143, 65-70.

6. Bostian, K. A., Lemire, J. M., Cannon, L. E. and Halvorson, H. O. (1980) Proc. Natl. Acad. Sci. USA 77, 4504-4508.

7. Kramer, R. A. and Andersen, N. (1980) Proc. Natl. Acad. Sci. USA 77, 6541-6545.

8. Toh-e, A., Ueda, Y., Kakimoto, S. and Oshima, Y. (1973) J. Bacteriol. 113, 727-738.

9. Lange, P. and Hansche, P.E. (1980) Molec. Gen. Genet. 180, 605-607.

10. Rogers, D. T., Lemire, J. M. and Bostian, K. A. (1982) Proc. Natl. Acad. Sci. USA 79, 2157-2161.

11. Stinchcomb, D. T., Struhl, K. and Davis, R. W. (1979) Nature 282, 39-43.

12. Hinnen, A., Hicks, J. B. and Fink, G. R. (1978) Proc. Natl. Acad. Sci. USA 75, 1929-1933.

13. Andersen, N., Thill, G. P. and Kramer, R. A. (1983) Molec. Cellular Biol. 3, 562-569.

14. Bostian, K.A., Hopper, J. E., Rogers, D. T. and Tipper, D. J. (1980) Cell 19, 403-414.

15. Familletti, P. C., Rubinstein, S. and Pestka, S. (1981) Methods in Enz. 78, 387-395.

16. McMaster, G. K. and Carmichael, G. G. (1977) Proc. Natl. Acad. Sci. USA 74, 4835-4838.

17. Thomas, P. S. (1980) Proc. Natl. Acad. Sci. USA 77, 5201-5205.

18. Thill, G. P., Kramer, R. A., Turner, K. J. and Bostian, K. A. (1983) Molec. Cellular Biol. 3, 570-579.

19. Bolivar, F., Rodriguez, R. L., Greene, P. L., Betlach, M. D., Heyneker, H. L., Boyer, H. W., Crosa, J. H. and Falkow, S. (1977) Gene 2, 95-113.

20. Broach, J. R. (1980) in The Molecular Biology of the Yeast Saccharomyces: Life Cycle and Inheritance, ed. Strathern, J. N., Jones, E. W. and Broach, J. R. (Cold Spring Harbor Laboratory, Cold Spring Harbor, NY) pp. 445-470.

21. Ueda, Y., Toh-e, A. and Oshima, Y. (1975) J. Bacteriol. 122, 911-922.

22. Holland, M. J. and Holland, J. P. (1978) Biochemistry 17, 4900-4907.

23. Ammerer, G., Hitzeman, R., Hagie, F., Barta, A. and Hall, B. D. (1981) in Recombinant DNA, Proceedings of the Third Cleveland Symposium on Macromolecules, ed. Walton, A. G. (Elsevier, Amsterdam) pp. 185-197.

24. Miyanohara, A., Toh-e, A., Nozaki, C., Hamada, F., Ohtomo, N. and Matsubara, K. (1983) Proc. Natl. Acad. Sci. USA 80, 1-5.

25. Hitzeman, R. A., Leung, D. W., Perry, L. J., Kohr, W.J., Levine, H. L. and Goeddel, D. V. (1983) Science 219, 620-625.

CLAIMS:

1. An expression vector comprising a DNA sequence, said sequence having a promoter/regulator DNA region derived from the yeast repressible acid phosphatase gene (PHO5) and a DNA coding for interferon.

2. The expression vector of claim 1 wherein the promoter/regulator DNA region is derived from the 5'-flanking region of the yeast repressible acid phosphatase gene.

3. The expression vector of claim 2 wherein the promoter/regulator is the PHO5 gene.

4. The expression vector of claim 1 wherein the interferon is human leukocyte interferon.

5. The expression vector of claim 1 wherein the interferon is recombinant human leukocyte interferon species A.

6. The expression vector of claim 1 wherein the interferon is recombinant human leukocyte interferon species D.

7. A host transformed with the expression vector according to any one of claims 1-6.

8. A host according to claim 7 wherein the host is a yeast organism.

9. A host according to claim 7 wherein the host is Saccharomyces cerevisiae.

10. A host according to claim 9 wherein interferon production can be induced by the absence of inorganic phosphate from the culture medium.

11. A host according to claim 9 wherein interferon production can be repressed by the presence of inorganic phosphate in the culture medium.

12. A transformed mutant strain of Saccharomyces cerevisiae, characterized in that it is transformed with an expression vector containing a heterologous eucaryotic DNA coding for a polypeptide operably linked to a promoter/-regulator DNA region derived from the yeast repressible acid phosphatase (PHO5) gene and wherein the repression and induction of the promoter/regulator and expression of the heterologous eucaryotic DNA is regulated only by temperature.

13. A host according to claim 12 wherein the promoter/-regulator and expression of the heterologous eucaryotic DNA is induced from about 20°C to about 30°C.

14. A host according to claim 13 wherein the promoter/-regulator is the PHO5 gene.

15. A host according to claim 14 wherein the heterologous eucaryotic DNA encodes for interferon.

16. A host according to claim 14 wherein the heterologous encaryotic DNA incodes for leukocyte interferon.

17. A host according to claim 16 wherein the leukocyte interferon is recombinant human leukocyte interferon species A.

18. A host according to claim 16 wherein the leukocyte interferon is recombinant human leukocyte interferon species D.

19. A method for producing interferon comprising:

a) transforming a yeast organism with an expression vector containing the structural gene for a human interferon operably linked to a PHO5 promoter/regulator;

b) cloning the transformed organism in a culture medium containing inorganic phosphate and essential nutrients for growth of the organism;

c) decreasing the levels of inorganic phosphate present in the culture medium until said transformed organism is induced to produce interferon;

d) lysing the transformed organism, and

e) recovering interferon from the resultant lysate.

20. The method of claim 19 wherein the yeast organism is Saccharomyces cerevisiae.

21. The method of claim 19 wherein the interferon is a human leukocyte interferon.

22. A method for producing interferon comprising:

a) transforming a temperature sensitive yeast organism with an expression vector containing the structural gene for a human interferon operably linked a PHO5 promoter/operator;

b) cloning the transformed organism in a culture medium at a temperature of about 30-35°C;

c) decreasing the temperature of the organism to about 20-30°C whereby interferon production is induced;

d) lysing the host organisms, and

e) recovering interferon from the resultant lysate.

23. The method of claim 22 wherein the temperature sensitive host organism is an acid phosphatase gene regulatory mutant strain of Saccharomyces cerevisiae.

ES 4100/35

24. The method of claim 22 wherein the interferon is a human leukocyte interferon.

CLAIMS:    (Austria)

1. A method for producing interferon comprising:

a) transforming a yeast organism with an expression vector containing the structural gene for a human interferon operably linked to a PHO5 promoter/regulator;

b) cloning the transformed organism in a culture medium containing inorganic phosphate and essential nutrients for growth of the organism;

c) decreasing the levels of inorganic phosphate present in the culture medium until said transformed organism is induced to produce interferon;

d) lysing the transformed organism, and

e) recovering interferon from the resultant lysate.

2. The method of claim 1 wherein the yeast organism is Saccharomyces cerevisiae.

3. The method of claim 1 wherein the interferon is a human leukocyte interferon.

4. A method for producing interferon comprising:

a) transforming a temperature sensitive yeast organism with an expression vector containing the structural gene for a human interferon operably linked a PHO5 promoter/operator;

b) cloning the transformed organism in a culture medium at a temperature of about 30-35°C;

c) decreasing the temperature of the organism to about 20-30°C whereby interferon production is induced;

d) lysing the host organism, and

e) recovering interferon from the resultant lysate.

EV 4100/35  **0124874**

5. The method of claim 4 wherein the temperature sensitive host organism is an acid phosphatase gene regulatory mutant strain of Saccharomyces cerevisiae.

6. The method of claim 4 wherein the interferon is a human leukocyte interferon.

7. The processes for the preparation of interferon as hereinbefore described, especially with reference to the examples.

***

Figure 1

Figures 2A and 2B

**A**

**B**

PHO5    AAGCAAATCGATTACCA A̲T̲G̲

pYE4-D   AAGCAAATCGCCGGGAATTC A̲T̲G̲

Figure 3

[confirmed by sequence analysis]

RAN 4100/35

Figure 4

0124874

Figure 5

RAN 4100/35

Figure 6

Figure 7

Figure 8

IFN-γD →  
G3PDH →

← 25S

← 17S

0   + 3 −   + 6 −   + 9 −   + 14.5 −   + 24 −

9/9

Figure 9